# EUROPEAN PATENT APPLICATION

(11) **EP 4 717 286 A1**
(43) Date of publication of application: **01.04.2026**
(21) Application number: 24202501.3
(22) Date of filing: 25.09.2024
(51) Int. Cl.: A61M 5/00

(54) **NEST WITH BOUNDED OPENINGS FOR LIFTING AND CENTRING OF SAID NEST**

(71) Applicant: SCHOTT Pharma Schweiz AG, 9001 St. Gallen (CH)
(72) Inventor: BERLINGER, Marcel, 9001 St. Gallen (CH); WALCHER, Ulrich, 9001 St. Gallen (CH)
(74) Representative: Schott Corporate IP

(57) **Abstract**

A structure comprising
a. a plurality of receptacles that are adapted and arranged for accommodating containers for pharmaceutical, medical and/or cosmetic compositions;
b. a first planar component that at least partially surrounds the plurality of receptacles, wherein the first planar component
i. has a maximum height that is less than or equal to 3 mm;
ii. comprises at least two openings of a first kind, wherein the following applies to each of said at least two openings of the first kind:
A. extends through the first planar component, and
B. comprises a boundary surface that extends around at least 80 % of a circumference of the respective opening of the first kind.

## Description

### FIELD OF THE INVENTION

The invention pertains to a structure for transporting a plurality of containers, wherein said containers are preferably for accommodating pharmaceutical, medical, and/or cosmetic compositions. The invention also pertains to a method for transporting a plurality of containers using the structure, and use of the structure for accommodating a plurality of containers.

### BACKGROUND

Containers, such as containers for pharmaceutical, medical and/or cosmetic compositions, are generally accommodated (e.g., held) and transported using structures known as nests, such as the nests commercially available from SCHOTT Pharma AG & Co. KGaA. The nests generally have openings or cavities for lifting said nests by inserting a finger and/or a tool in the openings or cavities. Said openings and cavities are often also used for centring the nest when containers accommodated in the nest are being filled. The presence of these openings and cavities has a number of disadvantages for a nest, such as reduced form stability and/or the requirement that more material is required for the nest. Structures for accommodating containers are disclosed in US 11819658 B2, WO 18198028 A1, EP 3653526 A1, EP 3535057 B1, EP 3413951 B1, and EP 3381828 A1.

### OBJECTS

An object of the present invention is to at least partially overcome at least one of the disadvantages encountered in the state of the art.

It is a further object of the invention to provide a structure (e.g., a nest) for the accommodation of containers, wherein said structure has a reduced deviation in the desired form, e.g., due to the production and/or the sterilisation of the structure.

It is a further object of the invention to provide a structure (e.g., a nest) for the accommodation of containers, wherein said structure has a reduced deformation under load.

It is a further object of the invention to provide a structure (e.g., a nest) for the accommodation of containers, wherein said structure requires less material to produce.

It is a further object of the invention to provide a structure (e.g., a nest) for the accommodation of containers, wherein said structure is easier to handle, e.g., the removal of structure from a tub. It is a further object of the invention to provide a structure (e.g., a nest) for the accommodation of containers, wherein said structure reduces the amount of liquid (e.g., water) that accumulated during a vapour sterilisation of the structure.

It is a further object of the invention to provide a structure (e.g., a nest) for the accommodation of containers, wherein less particles are formed when said structure is transported in a further structure (e.g., a tub).

### PREFERRED EMBODIMENTS OF THE INVENTION

A contribution to at least partially fulfilling at least one of the above-mentioned objects is made by any of the embodiments of the invention.
A 1^{st} embodiment of the invention is a structure comprising
a. a plurality of receptacles, wherein each receptacle of said plurality of receptacles
   i. is adapted and arranged for accommodating a container, preferably a container for accommodating a pharmaceutical, medical and/or cosmetic composition;
   ii. has a height of at least 10 mm, preferably at least 12 mm, and further preferably at least 15 mm,
   iii. has a first end and a further end, opposite the first end,
   iv. comprises a sidewall that at least partially encloses an interior of the receptacle;
b. a first planar component that at least partially surrounds, preferably surrounds, the plurality of receptacles, wherein the first planar component
   i. comprises a first face and a further face, opposite the first face, wherein
      A. the first face is directed towards the first ends of the plurality of receptacles, and
      B. the further face is directed towards the further ends of the plurality of receptacles,
   ii. has a maximum height that is less than or equal to 3 mm, preferably less than or equal to 2.5 mm, more preferably less than or equal to 2 mm, and further preferably less than or equal to 1.6 mm;
   iii. comprises at least two openings of a first kind, wherein the following applies to each of said at least two openings of the first kind:
      A. extends through the first planar component, and
      B. comprises a boundary surface that extends around at least 80 %, preferably at least 85 %, more preferably at least 90 %, and further preferably at least 95 % of a circumference of the respective opening of the first kind.

In a preferred embodiment of the structure, for each of the at least two openings of the first kind, the boundary extends around 100 % of the circumference of the respective opening of the first kind.

This preferred embodiment is a 2^{nd} embodiment of the invention, that preferably depends on the 1^{st} embodiment of the invention.

In a preferred embodiment of the structure, one of the following applies:
i. a maximum dimension of any further planar component that projects from the first planar component is 10 cm or less, preferably 8 cm or less, more preferably 6 cm or less, and further preferably 4 cm or less, wherein said maximum dimension is preferably measured along one or more directions that are parallel to the first face of the first planar component; or
ii. the structure comprises no further planar components that project from the first planar component.

This preferred embodiment is a 3^{rd} embodiment of the invention, that preferably depends on any of the 1^{st} to 2^{nd} embodiments of the invention.

In the 3^{rd} embodiment, if the structure comprises multiple further planar components, the maximum dimension as given in feature i. should be understood to apply to each of the further planar components.

In a preferred embodiment of the structure, each of the at least two openings of the first kind has a surface area that is at least 96 mm², preferably at least 100 mm², more preferably at least 110 mm², and further preferably at least 130 mm².

This preferred embodiment is a 4^{th} embodiment of the invention, that preferably depends on any of the 1^{st} to 3^{rd} embodiments of the invention.

In a preferred embodiment of the structure, each of the at least two openings of the first kind has a surface area that is in the range from 100 to 550 mm², preferably from 140 to 500 mm², more preferably from 180 to 480 mm² and further preferably from 220 to 450 mm².

This preferred embodiment is a 5^{th} embodiment of the invention, that preferably depends on any of the 1^{st} to 4^{th} embodiments of the invention.

In a preferred aspect of the 5^{th} embodiment, each of the at least two openings of the first kind has a surface area that is in the range from 200 to 500 mm², more preferably from 250 to 480 mm², even more preferably from 300 to 465 mm², further preferably from 350 to 450 mm², and particularly preferably 400 to 430 mm².

In a preferred embodiment of the structure, each of the at least two openings of the first kind has at least one or all of the following properties:
a. a maximum value for a first dimension, e.g., a length, that is at least 6 mm, preferably at least 8 mm, more preferably at least 10 mm, and further preferably at least 12 mm;
b. a maximum value for a second dimension, e.g., a width, that is at least 6 mm, preferably at least 8 mm, more preferably at least 10 mm, and further preferably at least 12 mm.

This preferred embodiment is a 6^{th} embodiment of the invention, that preferably depends on any of the 1^{st} to 5^{th} embodiments of the invention.

In an aspect of the 6^{th} embodiment, all possible combinations of the features a. and b. are preferred aspects of the embodiment. These combinations are *e.g.,* a; b; a+b.

In a preferred embodiment of the structure, each of the at least two openings of the first kind has at least one or all of the following properties:
a. a maximum value for a first dimension, e.g., a length, that is in the range from 10 to 40 mm, preferably from 12 to 35 mm, more preferably from 14 to 27 mm, and further preferably from 17 to 23 mm;
b. a maximum value for a second dimension, e.g., a width, that is in the range from 10 to 50 mm, preferably from 12 to 40 mm, more preferably from 14 to 30 mm, and further preferably from 16 to 25 mm.

This preferred embodiment is a 7^{th} embodiment of the invention, that preferably depends on any of the 1^{st} to 6^{th} embodiments of the invention.

In an aspect of the 7^{th} embodiment, all possible combinations of the features a. and b. are preferred aspects of the embodiment. These combinations are *e.g.,* a; b; a+b. In a preferred aspect of the 7^{th} embodiment, feature a., each of the at least two openings of the first kind each has a maximum value for the first dimension that is in the range from 18 to 20 mm, such as 19.05 mm.

In a preferred embodiment of the structure, the boundary surface of each of the at least two openings of the first kind has an average height of 14 mm or less, preferably 10 mm or less, more preferably 6 mm or less, and further preferably 4 mm or less.

This preferred embodiment is an 8^{th} embodiment of the invention, that preferably depends on any of the 1^{st} to 7^{th} embodiments of the invention.

In a preferred aspect of the 8^{th} embodiment, the average height of the boundary surface of each of the at least two openings of the first kind is in the range from 0.4 to 14 mm, preferably from 0.6 to 10 mm, more preferably from 0.8 to 6 mm, and further preferably from 1.0 to 4 mm.

In a preferred embodiment of the structure, the boundary surface of each of the at least two openings of the first kind has a maximum height of 14 mm or less, preferably 10 mm or less, more preferably 6 mm or less, and further preferably 4 mm or less.

This preferred embodiment is a 9^{th} embodiment of the invention, that preferably depends on any of the 1^{st} to 8^{th} embodiments of the invention.

In a preferred aspect of the 9^{th} embodiment, the maximum height of the boundary surface of each of the at least two openings of the first kind is in the range from 0.4 to 14 mm, preferably from 0.6 to 10 mm, more preferably from 0.8 to 6 mm, and further preferably from 1.0 to 4 mm.

In a preferred embodiment of the structure, the boundary surface is at least partially formed, preferably partially formed, by a first section.

This preferred embodiment is a 10^{th} embodiment of the invention, that preferably depends on any of the 1^{st} to 9^{th} embodiments of the invention.

In a preferred embodiment of the structure, the first section has a maximum value for a second dimension, e.g., a width, that in the range 0.5 to 11 mm, preferably from 1.0 to 9 mm, more preferably from 1.5 to 8 mm, and further preferably from 2 to 7 mm.

This preferred embodiment is an 11^{th} embodiment of the invention, that preferably depends on the 10^{th} embodiment of the invention.

In a preferred embodiment of the structure, a surface area of a cross-sectional cut of the first section is in the range from 0.1 to 40 mm², preferably from 0.5 to 30 mm², more preferably from 1 to 20 mm², and further preferably from 2 to 10 mm², wherein the cross-sectional cut is made
I. perpendicular to an edge of the first planar component, and
II. at a position where a second dimension, e.g., a width, of the first section has a minimum value.

This preferred embodiment is a 12^{th} embodiment of the invention, that preferably depends on any of the 10^{th} to 11^{th} embodiments of the invention.

In the 12^{th} embodiment, the cross-sectional cut preferably extends form the edge of the first planar component to the boundary surface of the opening of the first kind. In a preferred aspect of the 12^{th} embodiment, the surface area is in the range from 3 to 7 mm².

In a preferred embodiment of the structure, the first section is arranged parallel to a section of an edge of the first planar component.

This preferred embodiment is a 13^{th} embodiment of the invention, that preferably depends on any of the 10^{th} to 12^{th} embodiments of the invention.

In a preferred embodiment of the structure, one of the following applies:
a. the first section has a maximum height that is equal to or larger, preferably larger, than a maximum height of the first planar component; or
b. the first section has a maximum height that is equal to or less, preferably less, than a maximum height of the first planar component.

This preferred embodiment is a 14^{th} embodiment of the invention, that preferably depends on any of the 10^{th} to 13^{th} embodiments of the invention.

In a preferred embodiment of the structure, the maximum height of the first section is xH, where H is the maximum height of the first planar component and x is in the range from 0.1 to 10, preferably from 0.5 to 5, more preferably from 0.8 to 3, and further preferably from 1 to 2.

This preferred embodiment is a 15^{th} embodiment of the invention, that preferably depends on any of the 10^{th} to 14^{th} embodiments of the invention.

In a preferred embodiment of the structure, the maximum height of the first section is in the range from 0.4 to 14 mm, preferably from 0.6 to 10 mm, more preferably from 0.8 to 6 mm, and further preferably from 1.0 to 4 mm.

This preferred embodiment is a 16^{th} embodiment of the invention, that preferably depends on any of the 10^{th} to 15^{th} embodiments of the invention.

In a preferred embodiment of the structure, at least one or all of the following applies:
a. the maximum value of the first dimension, e.g., a length, of the first section is equal to or larger than the maximum value of the first dimension, e.g., the length, of the at least two openings of the first kind;
b. the maximum value of the first dimension, e.g., the length, of the first section is in the range from 3 to 70 mm, preferably from 10 to 50 mm, more preferably from 13 to 30 mm, and further preferably from 17 to 25 mm.

This preferred embodiment is a 17^{th} embodiment of the invention, that preferably depends on any of the 10^{th} to 16^{th} embodiments of the invention.

In an aspect of the 17^{th} embodiment, all possible combinations of the features a. and b. are preferred aspects of the embodiment. These combinations are *e.g.,* a; b; a+b.

In a preferred embodiment of the structure, the first section is integrally formed with the first planar component.

This preferred embodiment is an 18^{th} embodiment of the invention, that preferably depends on any of the 10^{th} to 17^{th} embodiments of the invention.

A 19^{th} embodiment of the invention is a method for transporting a plurality of containers, comprising the steps of
a. providing a structure according to the invention, preferably a structure according to any of the 1^{st} to 18^{th} embodiments of the invention;
b. receiving a plurality of containers in the structure, wherein said containers are preferably for accommodating a pharmaceutical, medical and/or cosmetic composition;
c. preferably transporting the structure and the plurality of containers from a first location to a further location.

In a preferred embodiment of the method for transporting a plurality of containers, the method further comprises at least one or all of the following steps:
A. inserting the structure into a further structure that at least partially encloses the structure, e.g., a tub;
B. sealing an opening of the further structure with a covering means, preferably a gas permeable covering means, preferably a planar covering means;
C. subjecting the structure and the containers received therein to a heat treatment, e.g., sterilisation, preferably while sealed in the further structure, e.g., the tub.

This preferred embodiment is a 20^{th} embodiment of the invention, that preferably depends on the 19^{th} embodiment of the invention.

In a preferred aspect of the 20^{th} embodiment, feature B., the covering means comprises a polymer, more preferably a non-woven polymer. Examples of a non-woven include a flash spun plexifilamentary film-fibril structure and a spunbonded very fine filaments non-woven. In this aspect it is preferred that the polymer is a polyolefin, more preferably a high-density polyethylene. A suitable covering means is available, e.g., under the trademark Tyvek^{®} from the company DuPont. An example of a covering means is a sealing lid.

A 21^{st} embodiment of the invention is a use of a structure according to the invention, preferably a structure according to any of the 1^{st} to 18^{th} embodiments of the invention, for accommodating a plurality of containers, wherein said containers are for accommodating a pharmaceutical, medical and/or cosmetic composition.

### DETAILED DESCRIPTION OF THE INVENTION

Features described as preferred in one category of the invention, for example according to the structure, are analogously preferred in an embodiment of the other categories according to the invention, such as a use of the structure according to the invention.

Throughout this document, disclosures of ranges should preferably be understood to include both end points of the range. Furthermore, each disclosure of a range in the document should preferably be understood as also disclosing preferred sub-ranges in which one end point is excluded or both end points are excluded. For example, a disclosure of a range from *X₁* to *X₂* is to be understood as disclosing a range that includes both of the end points *X₁* and *X₂.* Furthermore, it is to be understood as also disclosing a range that includes the end point *X₁* but excludes the end point *X₂,* a range that excludes the end point *X₁* but includes the end point *X₂,* and a range that excludes both end points *X₁* and *X₂.*

Some preferred embodiments and preferred aspects have various combinations of features as alternatives. Where these various combinations are disclosed, the combinations are separated by a semi-colon (";"). For example, the list of the combination of features "a; a+b; a+c+d" for a preferred embodiment discloses a preferred embodiment that comprises the feature "a", a preferred embodiment that comprises the features "a" and "b", and a preferred embodiment that comprises the features "a", "c", and "d".

### Structure

A structure preferably comprises a polymer. Examples of a preferred polymer include polypropylene, polyoxymethylene, polyethylene, polyethylene terephthalate, polystyrene, a cyclic olefin copolymer (COC) and a cyclic olefin polymer (COP). Here polypropylene is particularly preferred for the structure.

A structure may be obtained using deep drawing, moulding, preferably injection moulding, and/or 3D printing. A structure is preferably manufactured as a single piece. A structure with receptacles for accommodating containers, wherein said containers in turn are for accommodating pharmaceutical, medical and/or cosmetic compositions, is often referred to as a "nest". Here "nest" should preferably be understood as used in the pharmaceutical industry.

A structure preferably comprises 5 to 250, more preferably 10 to 200, even more preferably 20 to 190, further preferably 40 to 180, further preferably 60 to 180, further preferably 80 to 180, and even further preferably 100 to 160, receptacles.

A structure may be accommodated in a further structure known as a "tub". Here "tub" should preferably be understood as used in the pharmaceutical industry. The structure may be sealed with a covering means, such as a "sealing lid". Here "sealing lid" should preferably be understood as used in the pharmaceutical industry.

### Receptacle

A preferred receptacle is elongated. A first end of a receptacle preferably has a first opening for receiving a container. In other words, a receptacle is preferably adapted and arranged for a container to be inserted into and/or removed from said respective receptacle at the first end. The further end of a receptacle may be closed or may have a further opening. A further opening at the further end of a receptacle is preferably adapted and arranged to allow a part of a container to extend out of the receptacle when said container is accommodated in the receptacle. A preferred receptacle comprises at least one protrusion extending into an interior of said receptacle. A preferred receptacle has a height according to at least one norm and/or standard used in the pharmaceutical industry. In a preferred embodiment of the invention, the plurality of receptacles, of the first structure have heights of at least 17 mm, e.g., 17.5 mm.

### Protrusion

A protrusion is preferably adapted and arranged for positioning a container in a receptacle. For example, the protrusion is adapted and arranged for positioning the container during insertion of said container into a receptacle, and/or for positioning the container during removal of said container from a receptacle, and/or for positioning the container when said container is accommodated in a receptacle.

A protrusion preferably comprises, more preferably is made of, at least one thermoplastic. A protrusion may have any geometric shape. In a preferred aspect of the invention, one or more protrusions of a receptacle are formed as ribs. In a preferred aspect of the invention, a protrusion is formed as one piece with the sidewall of a receptacle. In a preferred aspect of the invention, a protrusion extends in a direction that is substantially perpendicular to the sidewall of the receptacle. In one preferred aspect of the invention, a protrusion and the sidewall, on which said protrusion is arranged, is made from the same material. In another preferred aspect of the invention, a protrusion and the sidewall, on which said protrusion is arranged, are made from different materials.

### First planar component

A first planar component is preferably arranged perpendicular to the plurality of receptacles. A first planar component may be arranged flush with first ends of the receptacles of a structure. A first planar component may be arranged flush with further ends of the receptacles of a structure. A first planar component may be arranged between first ends and further ends of the receptacles of a structure. A first planar component preferably comprises no sidewall extending around 100% of a circumference of the first planar component. If a first planar component comprises a sidewall partially extending around the circumference of the first planar component, it is preferred that the sidewall extends around 60 % or less, more preferably around 45 % or less, even more preferably around 30 % or less, and further preferably around 15 % or less of said circumference. A first planar component preferably comprises no sidewall partially extending around the circumference of the first planar component. A sidewall at least partially extending around the circumference of the first planar component is preferably arranged perpendicular to a first face of the first planar component.

### Further planar component

If a structure comprises a further planar component, the further planar component is preferably arranged within 10 mm, more preferably withing 6 mm, even more preferably within 3 mm, and further preferably within 1 mm from an outer boundary of the first planar component.

If a structure comprises a further planar component, an angle between the first planar component and the further planar component is preferably in the range from 30° and 150°, more preferably from 45° to 135°, even more preferably from 60° to 120°, further preferably from 75° to 105°, and even further preferably from 85° to 95°. If a structure comprises a further planar component, the angle between the first planar component and the further planar component is preferably 90°.

### Opening of the first kind

An opening of the first kind should preferably be understood as an opening that is adapted and arranged for facilitating a movement of a structure, to facilitate a positioning of a structure, or both. Example of a movement of a structure include the lifting from, and/or the inserting of a structure into, a further structure (e.g., a tub). Examples of a positioning of a structure is the centring of a structure during a filling of containers accommodated in said structure.

An opening of the first kind is preferably dimensioned for inserting at least one or all of the following: a finger, a component of a device adapted and arranged for moving a structure, a component of a device adapted and arranged for positioning a structure.

A preferred opening of the first kind is at least partially defined by a boundary surface. For example, the boundary surface of the opening of the first kind faces said opening. A boundary surface of an opening of the first kind may be a continuous surface or a discontinuous surface. A boundary surface of an opening of the first kind may be arranged in one or more geometrical planes.

An opening of the first kind having a circumference should not be understood to imply that the opening of the first kind has a circular cross-section. While the opening of the first kind may have a circular cross-section, this is not a requirement. In the present disclosure the term "circumference" should be understood to refer to a boundary for any shape of the opening of the first kind.

An opening of the first kind may be bounded or unbounded, provided the boundary surface extends around at least 80 % of a circumference of the opening of the first kind. It is, however, particularly preferred that the opening of the first kind is bounded. Here "unbounded" should be understood to mean that the boundary surface of the opening of the first kind does not extend around 100 % of the circumference of said opening of the first kind. Here "bounded" should be understood to mean that the boundary surface of the opening of the first kind extends around 100 % of the circumference of said opening of the first kind.

An opening of the first kind may have any shape that a skilled person deems suitable for the present invention. For example, the shape may be semi-circular, circular, triangular, square, rectangular, polygonal, tear-drop shaped, irregular, or a combination of at least two thereof.

A surface area of an opening of the first kind preferably does not vary by more than 30 %, more preferably by not more than 20 %, and further preferably by not more than 10 % along a height of opening of the first kind.

Examples of openings of the first kind are given in the standard ISO 11040-7. This standard ISO 11040-7 should not be interpreted as necessarily indicating or implying limitations to an opening of the first kind according to the invention. The standard ISO 11040-7 is provided as a reference and provides preferred values for some of the dimensions (e.g., a first dimension) of an opening of the first kind according to the invention.

### First section

A first section should preferably be understood as a section of a boundary surface of the opening of the first kind. A first section should preferably be understood as a section of the boundary surface, of the opening of the first kind, that is arranged closer to an edge of the first planar component than to a receptacle.

A first section is preferably formed as one piece with the first planar component. For example, the first section forms part of the first planar component. A first section is preferably straight. A first section preferably forms part of an edge of the first planar component. A face of a first section is preferably flush with an edge of the first planar component.

In one preferred embodiment of the invention, a first section has a unfirm height. In an alternative embodiment of the invention, the first section has a non-uniform height.

In one preferred embodiment of the invention, a maximum value of a first dimension of a first section is equal to a maximum value of a first dimension of an opening of the first kind. In an alternatively preferred embodiment of the invention, a maximum value of a first dimension of a first section is larger than a maximum value of a first dimension of an opening of the first kind.

### Containers

A container as referred herein comprises a container wall at least partially enclosing an interior volume of said container. A container as referred to herein is designed to accommodate a composition in such a way that an inner side of the container wall (i.e., the side of the container wall which faces the interior volume) and/or a coating layer (e.g., lubrication layer) on the inner side of the container wall can be in direct contact with the composition. A preferred container is suitable to be used for accommodating a pharmaceutical, medical, and/or cosmetic composition.

A first end of a container preferably comprises a first orifice, which allows for the charging of a composition into the interior volume of the container. Preferably, the first orifice also allows for the discharging of a composition from the interior volume.

A container may have any size or shape which the skilled person deems appropriate in the context of the invention. A preferred container has at least one section that is cylindrical. A preferred container is elongated.

A preferred container, more preferably the container wall, comprises, more preferably consists of, at least one glass, at least one polymer, or a combination thereof. A glass may be any type of glass and may have any composition which the skilled person deems suitable in the context of the invention. Preferably, the glass is suitable for pharmaceutical compositions. A preferred glass is of type I in accordance with the definitions of glass types in section 3.2.1 of the European Pharmacopoeia, 7^{th} edition from 2011. Examples of a preferred glass include borosilicate glass, fused silica, and combinations thereof. A particularly preferred glass is a borosilicate glass. Examples of a preferred polymer include polypropylene, polyoxymethylene, polyethylene, a cyclic olefin copolymer (COC) and a cyclic olefin polymer (COP). Here a cyclic olefin copolymer (COC) and a cyclic olefin polymer (COP) are particularly preferred.

Examples of preferred containers include cartridges, vials, ampules, carpules, and syringes.

In one preferred aspect of the invention, a container, more preferably a container wall, even more preferably a surface of the container wall facing the interior volume of the container, does not comprise a coating. In another preferred aspect of the invention, a container, more preferably the container wall, even more preferably a surface of the container wall facing the interior volume of the container, comprises at least one coating layer. Here a coating layer should preferably be understood as a layer that is applied by any method known to a skilled person working in the pharmaceutical industry. These methods include the application of a coating layer by spraying or wiping said coating layer on the container wall, as well as a coating layer is obtained by a plasma coating method. Examples of plasma coating methods include plasma impulse chemical vapour deposition and plasma-enhanced chemical vapour deposition.

A preferred coating layer comprises silicone, silane, or both. In a preferred aspect of the invention the coating layer is a lubrication layer. A preferred lubrication layer reduces friction when a component of the container is moved in the interior volume of the container. Here an example of a component is a plunger of a syringe, with the syringe being an example of a container. For example, a lubrication layer reduces the force required to move a plunger in a barrel of a syringe.

### Pharmaceutical and cosmetic composition

In the context of the invention, every pharmaceutical composition and every cosmetic composition which the skilled person deems suitable comes into consideration. A pharmaceutical composition is a composition comprising at least one pharmaceutically active ingredient. A preferred pharmaceutically active ingredient is a vaccine. A cosmetic composition is a composition comprising at least one cosmetically active ingredient. A preferred cosmetically active ingredient is hyaluronic acid or botulinum toxin. The pharmaceutical and/or cosmetic composition may be fluid or solid or both, wherein a fluid composition is particularly preferred herein. A preferred solid composition is granular such as a powder, a multitude of tablets or a multitude of capsules. A further preferred pharmaceutical and/or cosmetic composition is a parenterialium, i.e., a composition which is intended to be administered via the parenteral route, which may be any route which is not enteral. Parenteral administration can be performed injection, e.g. using a needle (usually a hypodermic needle) and a syringe, or by the insertion of an indwelling catheter.

### Dimensions

A first dimension is measured along a first direction. A second dimension is measured along a second direction, wherein said second direction is perpendicular to the first direction. A height is measured along a further direction, wherein said further direction is perpendicular to both the first direction and the second direction.

### TEST METHODS

Values for a first dimension, a second dimension, a height are measured using a vernier calliper. Values for a maximum dimension of a further planar component is also measured using a vernier calliper.

An average height of a boundary surface of an opening of the first kind is determined by measuring the height of the boundary surface at 3 mm intervals along the boundary surface. An average height is then calculated from these measurements. If a first section is present, height measurements should also be made on the first section, with these measurements forming part of the calculation of the average height. However, if no first section is present, then the height measurements are only made where the boundary surface is present.

A surface area of a cross-sectional cut of a first section is determined using a 3D scan device, such as a model VL-700 commercially available from Keyence Corporation (Japan). A surface area of an opening of the first kind may also be determined using the 3D scan device.

The invention is now illustrated by non-limiting examples and exemplifying embodiments.

### FIGURES

### List of figures

The figures serve to exemplify the present invention and should not be viewed as limiting the invention. The figures are not drawn to scale. Wording such as "above", "below", or similar, used in the figure descriptions, should be understood in the context of the figures.
Fig. 1A: schematic illustration of the structure according to the invention, viewed from above.
Fig. 1B: schematic illustration of the structure shown in Fig. 1A, viewed from the side.
Fig. 2A: schematic illustration showing how dimensions of an opening of the first kind are measured.
Fig. 2B: schematic illustration showing how height is measured.
Fig. 3A: schematic illustration showing a section of a structure according to the invention.
Fig. 3B: schematic illustration showing a section of a structure not according to the invention.
Figs 4A and 4B: variations of a structure according to the invention.
Figs 5A to 5C: schematic illustrations showing alternative shapes for an opening of the first kind, also according to the invention.
Figs 6A to 6F: schematic illustrations showing various alternative embodiments of a first section, also according to the invention.
Figs 7A to 7C: schematic illustrations showing further aspects of the invention related to an opening of the first kind.
Fig. 8: schematic illustration showing a section of a structure not according to the invention.
Figs 9A, 9B, 10A, and 10B: simulations comparing stresses in structures according to the invention to stresses in structures not according to the invention.

### Description of figures

### The figures are described in more detail below

### Figs 1A and 1B

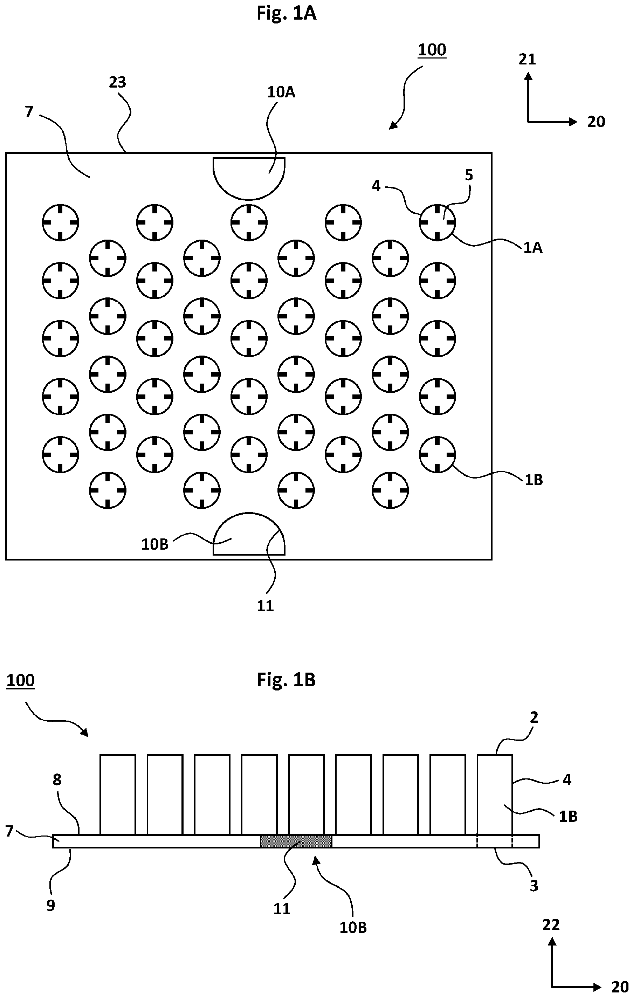

Fig. 1A is a schematic illustration of a structure 100 according to the invention, viewed from above. The structure 100 comprises a plurality of receptacles, such as 1A and 1B. The receptacles are adapted and arranged for accommodating containers, wherein said containers in turn are for accommodating a pharmaceutical, medical and/or cosmetic composition. As shown for receptacle 1A, the receptacles comprise sidewalls 4 that at least partially encloses interiors 5 of the receptacles. The structure 100 further comprises a first planar component 7 that surrounds the plurality of receptacles. The first planar component 7 has an edge 23, or boundary.

The structure 100 also comprises two openings of the first kind 10A and 10B. The openings of the first kind 10A and 10B are arranged in the first planar component 7 and extend through the first planar component 7. The openings of the first kind 10A and 10B are adapted and arranged for inserting a finger and/or a component of a device into said openings to, e.g., lift the structure 100 out of a tub, and/or to position the structure 100 during a filling of containers accommodated in the structure 100. As shown for opening 10B, the openings of the first kind comprise boundary surfaces 11 that extend around 100 % of the circumferences of said openings. It can also be seen that the openings of the first kind 10A and 10B are arranged symmetrically.

Fig. 1B is a schematic illustration of the structure 100 shown in Fig. 1A, viewed from the side. Fig. 1B shows that the first planar component 7 has a first face 8 and a further face 9 (opposite the first face 8). As shown for receptacle 1B, the first face 8 is directed to the first ends 2 of the receptacles, while the further face 9 is directed to the further ends 3 of the receptacles. Containers are inserted into, and removed from, the receptacles at the first ends 2.

### Figs 2A and 2B

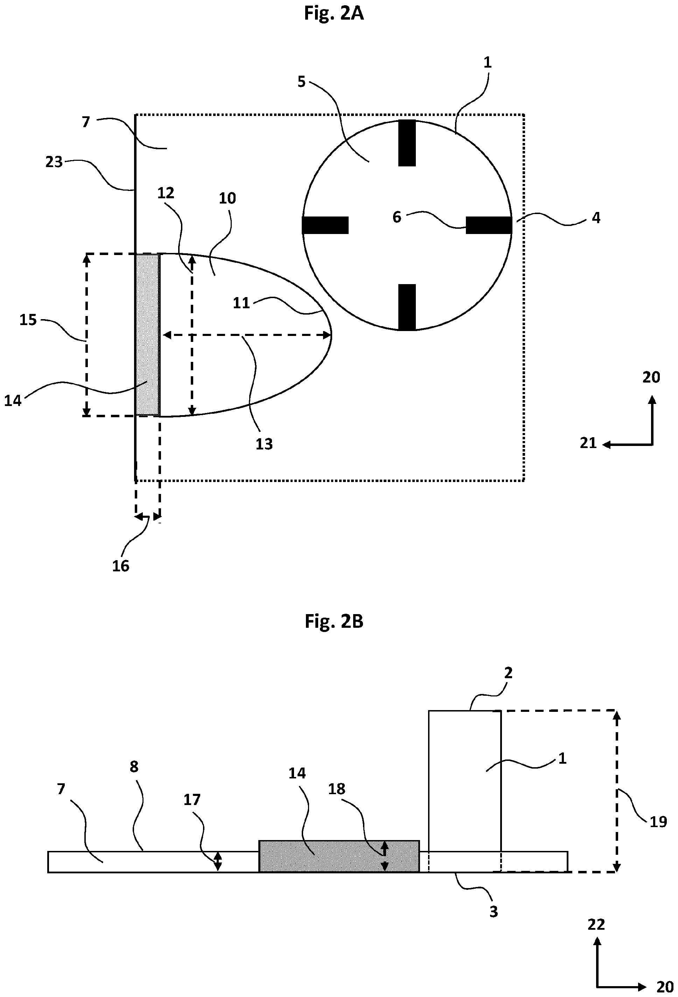

Fig. 2A is a schematic illustration showing how the dimensions of an opening of the first kind are measured (Fig. 2A shows a section of a structure according to the invention). A first dimension 12 (e.g., a length) of the opening of the first kind 10 is measured along a first direction 20, wherein said first direction 20 is arranged parallel to the edge 23 of the first planar component 7. A second dimension 13 (e.g., a width) of the opening of the first kind 10 is measured along a second direction 21, wherein said second direction 21 is perpendicular to the first direction 20.

Fig. 2A also shows that the boundary surface 11 of the opening of the first kind 10 is partially formed by a first section 14. Said first section 14 is arranged parallel to the edge 23 of the first planar component 7. A first dimension 15 (e.g., a length) of the first section 14 is measured along the first direction 20, while a second dimension 16 (e.g., a width) of the first section 14 is measured along the second direction 21.

Fig. 2A also shows that the receptacle 1 comprises protrusions 6 that extend into the interior 5 of the receptacle 1. Said protrusions 6 allow for an improved positioning of a container in the receptacle 1.

Fig. 2B is a schematic illustration showing how height is measured. Fig. 2B shows a structure according to the invention, viewed from the side (features of the structure, e.g., shown in Fig. 1B, have been omitted for the purposes of illustration). As shown in Fig. 2B, a height 17 of the first planar component, a height 18 of the first section 14, and a height 19 of the receptacle 1 are measured along a further direction 22. Although not shown in Fig. 2B, a height of the boundary surface of the opening of the first kind is also measured along the further direction 22. Said further direction 22 is perpendicular to both the first direction 20 and the second direction 21, and thus perpendicular to, e.g., the first face 8 of the first planar component. If the receptacle 1 has a non-uniform cross-section (e.g., the receptacle 1 is conical), the height 19 is still measured along the further direction 22, between the first end 2 and the second end 3 of the receptacle 1.

### Figs 3A and 3B

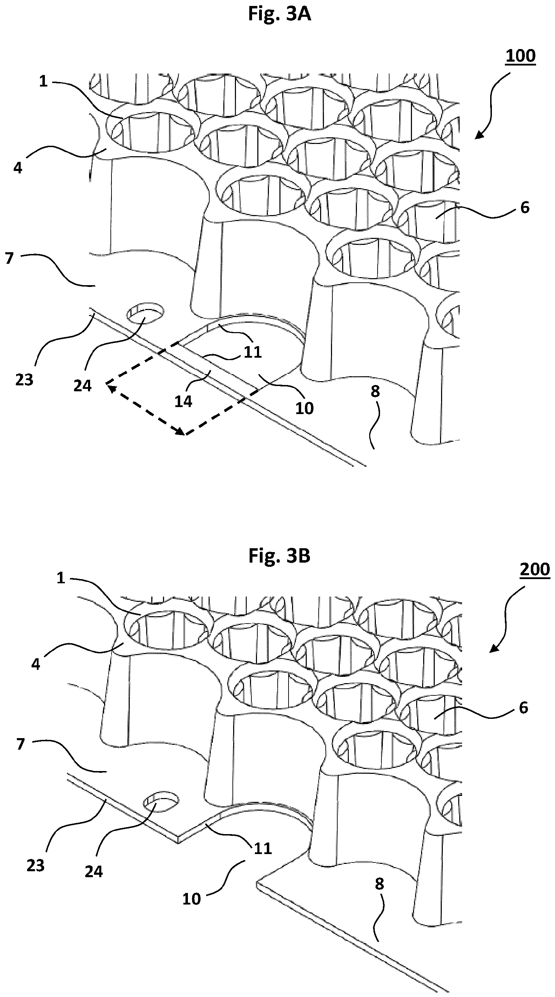

Fig. 3A is a schematic illustration showing a section of a structure 100 according to the invention, while Fig. 3B is a schematic illustration showing a section of a structure 200 not according to the invention. Fig. 3A shows that the boundary surface 11 of the opening of the first kind 10 extends around 100 % of a circumference of said opening. The opening of the first kind 10 in Fig. 3A is thus bounded. The boundary surface 11 is partially formed by the first section 14 (located between the dashed lines in Fig. 3A). As also shown in Fig. 3A, the receptacles 1 have protrusions 6 that extend into the interiors of said receptacles 1. Furthermore, these protrusions 6 are formed as ribs that extend along a height of the receptacles 1.

The structure 200 in Fig. 3B is similar to the structure 100 in Fig. 3A, with the following difference. In contrast to Fig. 3A, the opening of the first kind 10 in Fig. 3B has a boundary surface 11 that extends around less than 80 % of a circumference of said opening. This is due to the absence of a first section. As the boundary surface 11 of the opening of the first kind 10 in Fig. 3B does not extend around the circumference of said opening, the opening of the first kind in Fig. 3B is unbounded.

Figs 3A and 3B show that a structure may comprise an opening of a further kind 24. In contrast to the opening of the first kind 10, the opening of the further kind 24 is not adapted and arranged for e.g., lifting the structure from a further structure (e.g., a tub). In other words, the opening of the further kind 24 is not adapted and arranged for inserting a finger in said opening.

### Figs 4A and 4B

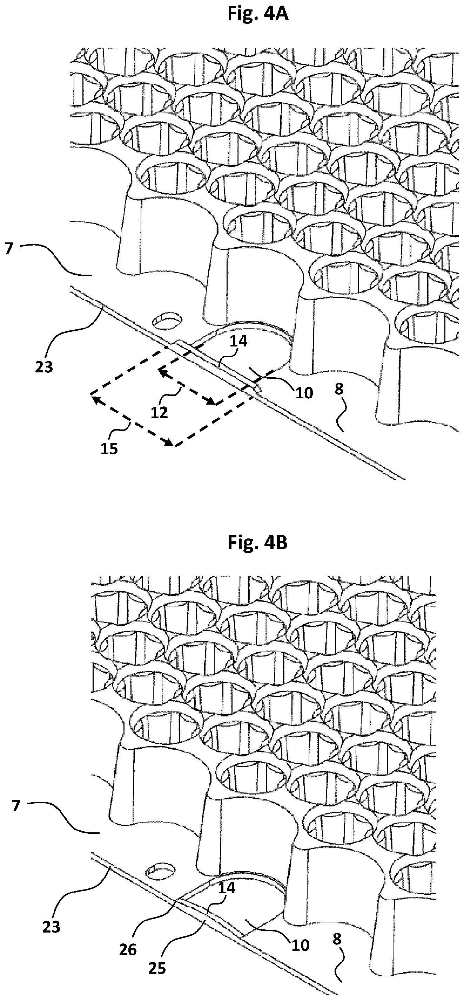

Figs 4A and 4B show variations of a structure according to the invention. As shown in Fig. 4A, the first section 14 has a first dimension 15 that is larger than the first dimension 12 of the opening of the first kind 10. It can also be seen that the first section 14 is raised (i.e., a height of the first section 14 is larger than a height of the first planar component).

Fig. 4B also shows a first section 14 with a height that is larger than the height of the first planar component 7. The height of the first section 14 also varies between a centre 25 and an end 26 of the first section 14. The height of the first section 14 is measured at the point where said height is the largest. In Fig. 4B, this is at the centre 25. From Fig. 4B it can also be seen that the first section 14 has a first dimension 15 that is equal to the first dimension 12 of the opening of the first kind 10.

The first dimension and the height of a first section are independent features. For example, the first section in Fig. 4B may also have a first dimension that is larger than the first dimension of the opening of the first kind.

### Figs 5A to 5C

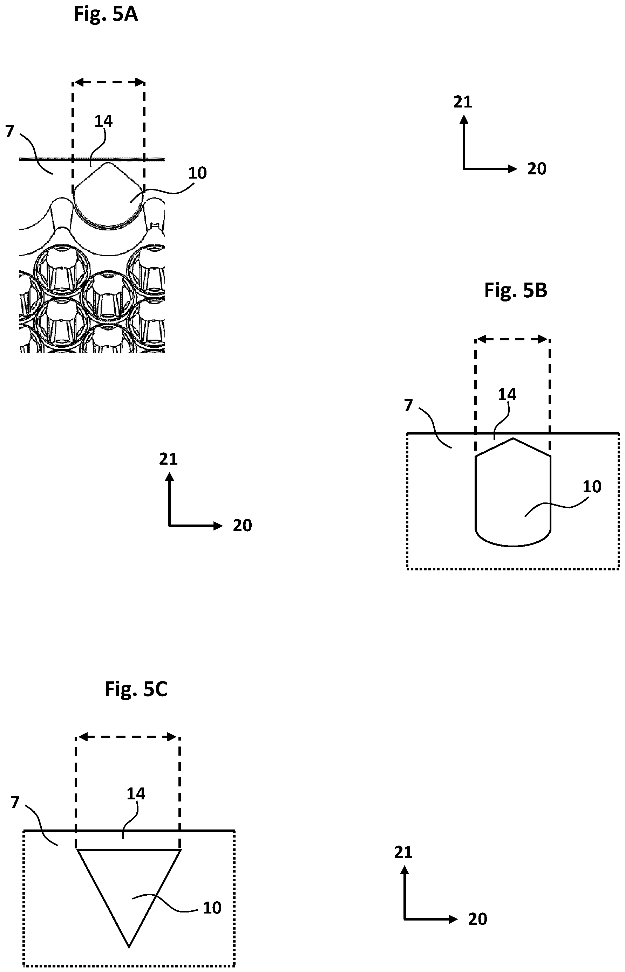

Figs 5A to 5C are schematic illustrations showing alternative shapes for an opening of the first kind 10, also according to the invention. The openings of the first kind 10 in Figs 5A to 5C are viewed from above. In Figs 5A to 5C the first section 14 is the part of the first planar section that is located between the dashed lines.

### Figs 6A to 6F

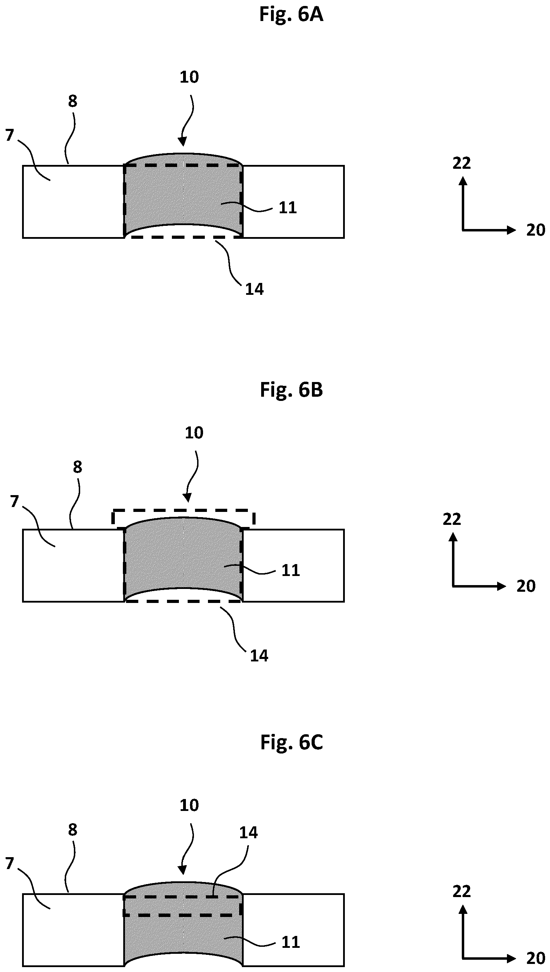

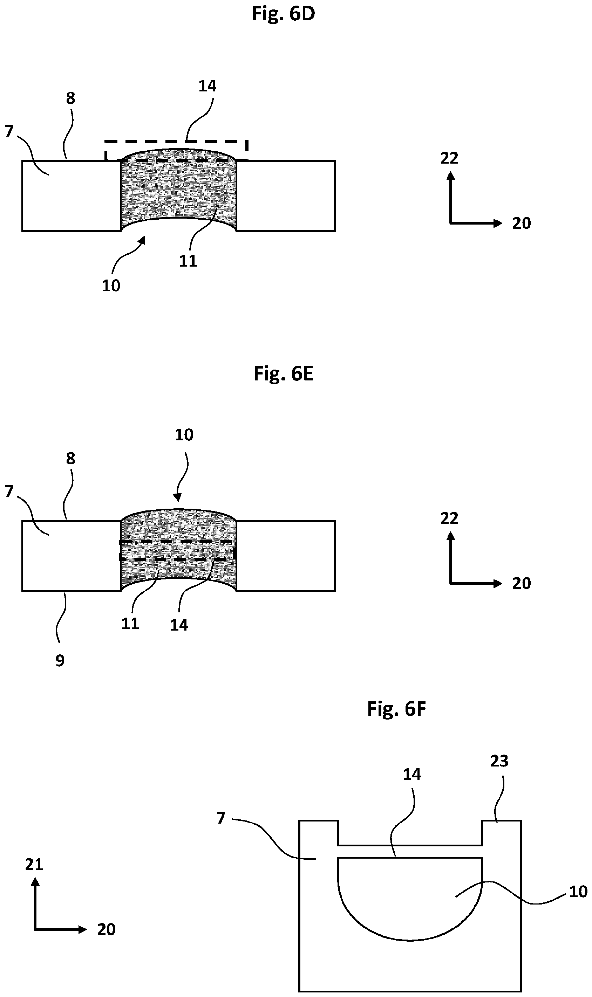

Figs 6A to 6F are schematic illustrations showing various alternative embodiments of a first section 14, also according to the invention. Figures 6A to 6E show a section of a structure viewed from the side, with the first sections 14 indicated by the dashed area. Fig. 6F show an opening of the first kind 10 viewed from above.

The first section 14 in Fig. 6A is similar to the first section in, e.g., Fig. 3A. The first section in Fig. 6B is similar to the first section in, e.g., Fig. 4A. As shown in Figs 6C to 6E, a height of the first section 14 (measured along the further direction 22) can be less than a height of the first planar component 7. Furthermore, the first section 14 may be arranged flush with a first face 8 of the first planar component 7 (Fig. 6C), may be arranged above the first face 8 (Fig. 6D), or may be arranged between the first face 8 and a further face 9 of the first planar component 7 (Fig. 6E).

Alternative embodiments of the first section not shown in Figs 6A to 6E may also be possible. For example, the first section may have a height that is less than a height of the first planar component, with the first section arranged flush with the further face of the first planar component (i.e., the opposite of Fig. 6C). Similarly, the first section may also be arranged similar to the first section shown in Fig. 6D, but with the first section arranged below the further face of the first planar component.

Each of the openings of the first kind 10 in Figs 6A to 6E are bounded, i.e., the boundary surface extends around 100 % of a circumference of said openings. Noting that the first section 14 forms part of the boundary surface, it can be seen in, e.g., Fig. 6D that the boundary surface 11 can be arranged in different geometric planes. In Fig. 6D the first section 14 is arranged above the remaining sections of the boundary surface 11. This boundary surface 11 in Fig. 6D is also discontinuous.

Figs 6A to 6E also illustrate how a maximum height of a boundary surface of an opening of the first kind should be measured. It should be noted that a surface of the first section 14 that faces the opening of the first kind forms part of the boundary surface. In Fig. 6A, the boundary surface has a uniform height, and the maximum height can be measured at any position on the boundary surface (including at the first section 14). In Fig. 6B, the height of the first section 14 is larger than the height measured at other positions of the boundary surface. The maximum height of the boundary surface is thus measured at the first section 14. In Figs 6C to 6E, the first section 14 has a height that is less than the height of other parts of the boundary surface. The maximum height of the boundary surface will thus not be measured at the first section 14. As indicated, the maximum height of the boundary surface should thus be measured at the position where the height is the largest.

Fig. 6F show an opening of the first kind 10 where the first section 14 is not arranged flush with an edge 23 of the first planar component 7 (the first section 14 in Fig. 6F is arranged parallel to the edge 23). Alterative embodiments of the first section 14 shown in Fig. 6F, also according to the invention, are also possible. For example, the first section 14 in Fig. 6F may be arranged at an angle to the edge 23. Combinations of the embodiment shown in Fig. 6F may also be combined with, e.g., the embodiments shown in Figs 6A to 6E.

### Figs 7A to 7C

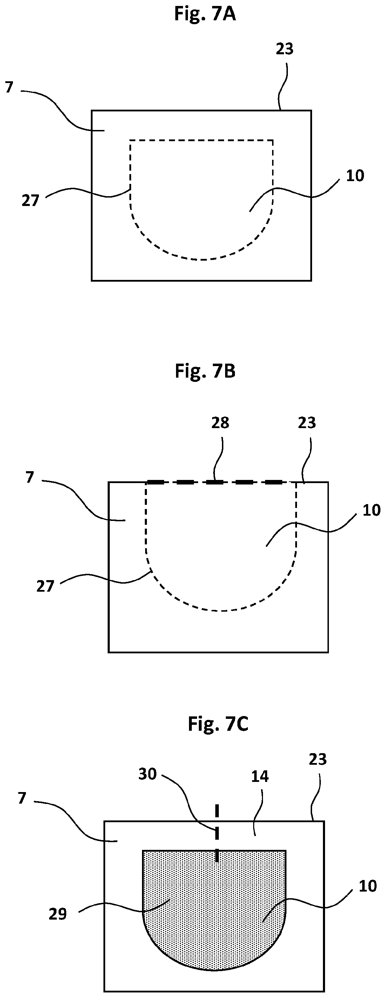

Figs 7A to 7C are schematic illustrations showing further aspects of the invention related to an opening of the first kind. Figs 7A to 7C show openings of the first kind viewed from above.

Fig. 7A shows a circumference 27 of a bounded opening of the first kind 10, indicated by the dashed line (the opening 10 in Fig. 7A is similar to the opening 10 shown in Fig. 3B). As can be seen, the circumference 27 extends around said opening of the first kind 10. Fig. 7B shows an unbounded opening of the first kind 10 (such as shown in Fig. 3B). For the opening of the first kind 10 in Fig. 7B, the circumference 27 of said opening 10 is defined by drawing an imaginary line 28 along the edge 23 of the first planar component, as shown in Fig. 7B. This imaginary line 28 is then considered to form part of the circumference 27 of the opening of the first kind 10.

Fig. 7C shows how a surface area 29 of an opening of the first kind 10 is defined. The surface area 29 is indicated by the hatched area. The hatched area also indicates the shape 29 of the opening of the first kind 10. Fig. 7C also shows how the surface area of a cross-sectional cut of the first section 14 is defined. The cross-sectional cut should be made at the position 30 indicated by the dashed line. Said dashed line is perpendicular to an edge 23 of the first planar component 7.

### Fig. 8

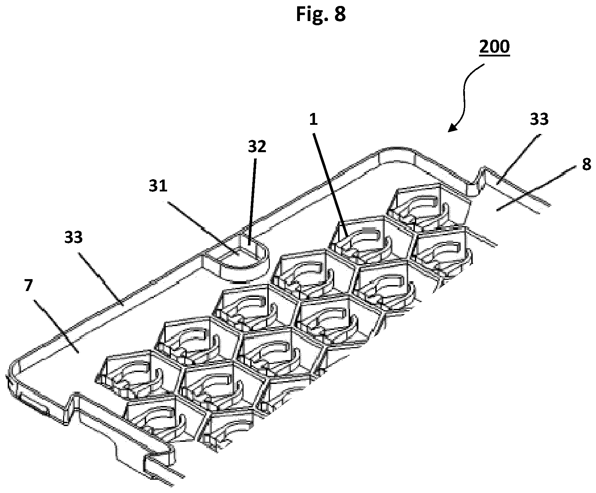

Fig. 8 is a schematic illustration showing a section of a structure 200 not according to the invention. As can be seen in Fig. 8, the structure 200 has an opening of the first kind 31. Comparable to an opening of the first kind 10, the opening of the first kind 31 is used for inserting a component of a device for lifting the structure. Fig. 8 shows that the opening of the first kind 31 is partially surrounded by opening walls 32 that also form a boundary surface of the opening 31. Said opening walls 32 project from the first planar surface 7. A height of the boundary surface of the opening of the first kind 31 should be understood to include the height of the opening walls 32 (the height of the opening walls is also measured along the further direction). In other words, a maximum height of the boundary surface of the opening of the first kind 31 in Fig. 8 is larger than the height of a boundary surface of a similar opening of the first kind without the opening walls 32.

Fig. 8 also shows a further planar component 33 projecting from the first planar component 7. The further planar component 33 in Fig. 8 forms a sidewall that extends around 100 % of a circumference of the first planar component 7. In Fig. 8, a maximum dimension of the further planar component 33 is measured along one or more directions that are parallel to the first face 8 of the first planar component 7. The maximum dimension of the further planar component 33 is not necessarily a dimension (e.g., length) of the further planar component 33 measured along a single direction. Rather, in Fig. 8 the maximum dimension of the further planar component 33 is a total length of said component 33 measured around a circumference of the first planar component 7. If a structure has a further planar component, a maximum height of the first planar component should be understood to include the height of the further planar component (the height of the further planar component is also measured along the further direction). In other words, the maximum height of the first planar component 7 in Fig. 8 is larger than the height of a first planar component of a similar structure without the further planar component 33.

If the opening 31 in Fig. 8 did not extend through the first planar component 7, but, e.g., was formed by sidewalls 32 arranged on the first planar component 7 such that said planar component formed a bottom surface of the opening 31, this would be an example of a cavity.

### EXAMPLES

The invention is illustrated further by way of examples. The invention is not restricted to the examples.

### Example 1

In Example 1, three different types of nests (structures) for accommodating containers for pharmaceutical, medical and/or cosmetic compositions are provided.
- Example 1.1: a nest, according to the invention, that comprises bounded openings of the first kind, as shown in Fig. 3A.
- Example 1.2: a nest, not according to the invention, that comprises unbounded openings of the first kind, as shown in Fig. 3B.
- Example 1.3: a nest, not according to the invention, that comprises openings of the first kind with sidewalls, as shown in Fig. 8.

Table 1 shows a comparison of the nests of Examples 1.1 to 1.3.

**Table 1**

| **Example** | **1.1** (inventive) | **1.2** (comparative) | **1.3** (comparative) |
|---|---|---|---|
| | | | |

| **Set-up** | | | |
|---|---|---|---|
| Openings of the first kind / cavities | Bounded openings | Unbounded openings | Bounded openings with sidewalls |
| | | | |

| **Technical effects** | | | |
|---|---|---|---|
| Deviation in desired form of nest | -- | + | -- |
| Deformation of nest under load | -- | ++ | --- |
| Material required for nest | - | -- | ++ |
| Ease or handling nest | ++ | ++ | - |
| Long-term form stability | + | --- | + |
| Accumulation of water during sterilisation of nest | -- | - | +++ |
| Particle generation during transport inside tub | --- | - | ++ |

The technical effects in Table 1 are as follows:
- Deviation in desired form of nest: the degree to which the nest deviates from the desired form during production or sterilisation of the nest. A "-" indicates less deviation and a "+" indicates more deviation. It is desired to decrease the deviation from the desired from.
- Deformation of nest under load: the degree to which the nest deforms when subjected to a load and/or stresses. A "-" indicates less deformation and a "+" indicates more deformation. It is desired to decrease the deformation of the nest.
- Material required for nest: the amount of material required to produce a nest that can withstand a certain amount of stress without deformation. A "-" indicates that less material is required and a "+" indicates that more material is required. It is desired to reduce the amount of material required.
- Ease of handling of nest: how easy it is to handle the nest, including removal of the nest from a tub, and the ease of access to the openings of the first kind / cavities by robotic suction tools. A "-" indicates a reduced ease of handling and a "+" indicates an increased ease of handling. It is desired to increase the ease of handling.
- Long-term form stability: nests under load (e.g., due to containers being accommodated in the nests) are subject to material creep. Continual use and storage of the nests over extended periods (e.g., more than a year) leads to deformation of the nests. A "+" indicates that a nest is more form-stable (i.e., undergoes less deformation) while a "-" indicates that a nest is less form-stable (i.e., undergoes more deformation). It is desired to have an increased form stability.
- Accumulation of water during sterilisation of the nest: the amount of water that accumulates on the nest when subjected to vapour sterilisation. For example, a nest with sidewalls leads to the accumulation of water on the first planar component, while a nest that deforms also leads to the accumulation of water. A "-" indicates less water accumulation and a "+" indicates more water accumulation. It is desired to reduce the water accumulation.
- Particle generation during transport inside tub: the number of particles that form while the nest is stored and/or transported in a tub. This particle formation occurs due to, e.g., the rubbing of the nest on the surfaces of the tub where the nest contacts the tub (e.g., the surfaces of the tub on which the nest rests). A "+" indicates more particle formation while a "-" indicates less particle formation. It is desired to reduce particle formation.

### Example 2

In Example 2 the deformation of nests was investigated using finite element simulations. The receptacles of the nests have protrusions that extend into interiors of the receptacles (as shown in, e.g., Fig. 3A and 3B). These protrusions are in the form or ribs that also extend along a height of the receptacles. In example 2, the heights of these ribs were varied

Nest with bounded openings of the first kind are according to the invention and are as shown in Fig. 3A. Nest with unbounded openings of the first kind are not according to the invention and are as shown in Fig. 3B.

The results of Example 2 are shown in Table 2.

**Table 2**

| | | | | |
|---|---|---|---|---|
| **Example** | **2.1** (inventive) | **2.2** (comparative) | **2.3** (inventive) | **2.4** (comparative) |
| | | | | |

| **Set-up** | | | | |
|---|---|---|---|---|
| Openings of the first kind | Bounded | Unbounded | Bounded | Unbounded |
| Height of ribs [mm] | 4 | 4 | 7 | 7 |
| Results shown in Fig. | 9A | 9B | 10A | 10B |
| | | | | |

| **Technical effects** | | | | |
|---|---|---|---|---|
| Centre deflection [mm] | 0.47 | 0.63 | 0.25 | 0.30 |
| Von Mises stress at centre [MPa] | 1.09 | 1.67 | 0.90 | 1.04 |
| Von Mises stress at opening of the first kind [MPa] | 0.75 | 2.24 | 0.12 | 1.27 |

The technical effects in Table 2 are as follows:
- Centre deflection: the deformed nests form a depression in the centre of the nest. The deflection is the depth of this depression.
- Von Mises stress at centre: the stresses as measured at the centre of the nest.
- Von Mises stress at opening of the first kind: the stresses as measured at the opening of the first kind.

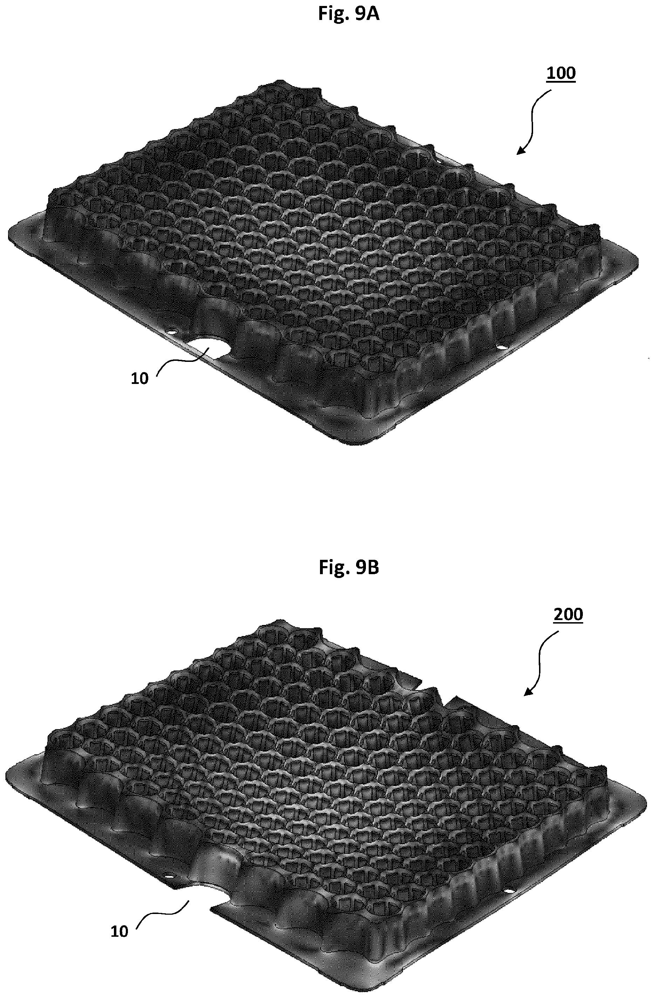

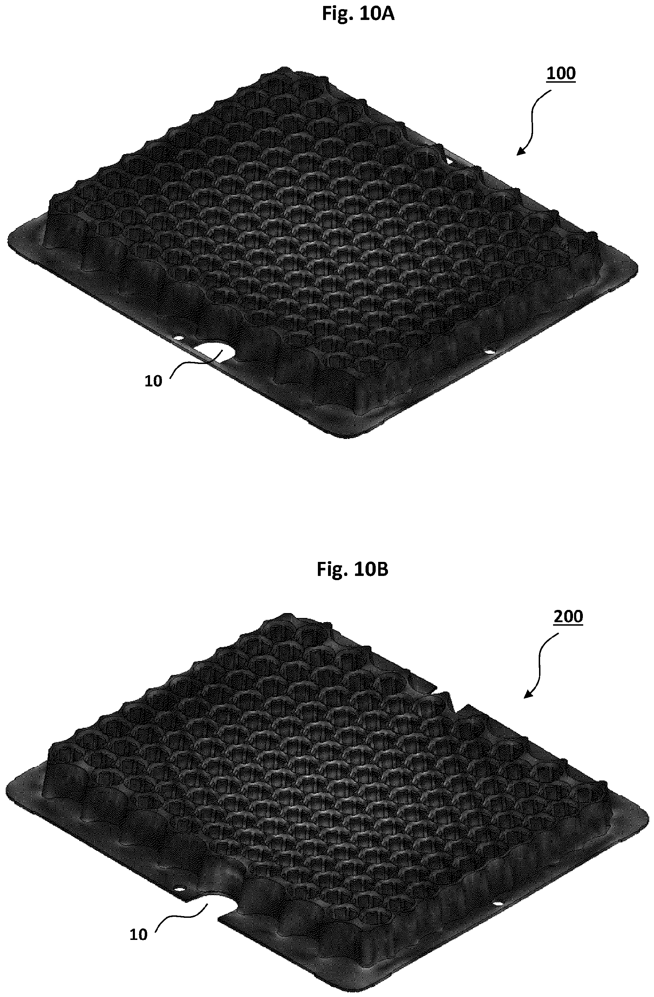

The results of the simulations are shown in Figs 9A, 9B, 10A and 10B. Structures 100 in Figs 9A and 10A are according to the invention, while structures 200 in Figs 9B and 10B are not according to the invention. Lighter areas in the structures indicate regions with a larger von Mises value (i.e., larger stresses). In these figures it can be seen that depressions are formed in the centre of the structures. The simulations results show that a bounded opening of the first leads to a significant reduction in the deformation of a structure, compared to an unbounded opening.

Comparing Figs 9A and 9B, it can be seen that structure 100 has less stresses in the centre of said structure comparted to structure 200. The depression formed at the centre of structure 100 is also shallower compared to the depression formed in structure 200. Similar results are also obtained for the structures 100 and 200 in Figs 10A and 10B (however, compared to the structures in Figs 9A and 9B, the structures in Figs 10A and 10B have less deformation).

### REFERENCE LIST

- 1: Receptacle
- 2: First end of receptacle
- 3: Further end of receptacle
- 4: Sidewall of receptacle
- 5: Interior of receptacle
- 6: Protrusion of receptacle
- 7: First planar component
- 8: First face of first planar component
- 9: Further face of first planar component
- 10: Opening of the first kind
- 11: Boundary surface of opening of the first kind
- 12: First dimension of opening of the first kind
- 13: Second dimension of opening of the first kind
- 14: First section
- 15: First dimension of first section
- 16: Second dimension of first section
- 17: Height of first planar component
- 18: Height of first section
- 19: Height of receptacle
- 20: First direction
- 21: Second direction
- 22: Further direction
- 23: Edge of first planar component
- 24: Opening of a further kind
- 25: Centre of first section
- 26: End of first section
- 27: Circumference of opening of the first kind
- 28: Imaginary line
- 29: Surface area / shape of opening of the first kind
- 30: Position of cross-sectional cut
- 31: Opening of the first kind
- 32: Opening wall
- 33: Further planar component

## Claims

1. A structure comprising
a. a plurality of receptacles, wherein each receptacle of said plurality of receptacles
i. is adapted and arranged for accommodating a container;
ii. has a height of at least 10 mm,
iii. has a first end and a further end, opposite the first end,
iv. comprises a sidewall that at least partially encloses an interior of the receptacle;
b. a first planar component that at least partially surrounds the plurality of receptacles, wherein the first planar component
i. comprises a first face and a further face, opposite the first face, wherein
A. the first face is directed towards the first ends of the plurality of receptacles, and
B. the further face is directed towards the further ends of the plurality of receptacles,
ii. has a maximum height that is less than or equal to 3 mm;
iii. comprises at least two openings of a first kind, wherein the following applies to each of said at least two openings of the first kind:
A. extends through the first planar component, and
B. comprises a boundary surface that extends around at least 80 % of a circumference of the respective opening of the first kind.

2. The structure according to claim 1, wherein, for each of the at least two openings of the first kind, the boundary extends around 100 % of the circumference of the respective opening of the first kind.

3. The structure according to any of the preceding claims, wherein one of the following applies:
i. a maximum dimension of any further planar component that projects from the first planar component is 10 cm or less; or
ii. the structure comprises no further planar components that project from the first planar component.

4. The structure according to any of the preceding claims, wherein each of the at least two openings of the first kind has a surface area that is in the range from 100 to 550 mm².

5. The structure according to any of the preceding claims, wherein each of the at least two openings of the first kind has at least one or all of the following properties:
a. a maximum value for a first dimension that is in the range from 10 to 40 mm;
b. a maximum value for a second dimension that is in the range from 10 to 50 mm.

6. The structure according to any of the preceding claims, wherein at least one or all of the following applies:
a. the boundary surface of each of the at least two openings of the first kind has an average height of 14 mm or less;
b. the boundary surface of each of the at least two openings of the first kind has a maximum height of 14 mm or less.

7. The structure according to any of the preceding claims, wherein the boundary surface is at least partially formed by a first section, wherein the first section has a maximum value for a second dimension that in the range 0.5 to 11 mm.

8. The structure according to any of the preceding claims, wherein the boundary surface is at least partially formed by a first section, wherein a surface area of a cross-sectional cut of the first section is in the range from 0.1 to 40 mm², wherein the cross-sectional cut is made
I. perpendicular to an edge of the first planar component, and
II. at a position where a second dimension, e.g., a width, of the first section has a minimum value.

9. The structure according to any of the preceding claims 7 to 8, wherein the first section is arranged parallel to a section of an edge of the first planar component.

10. The structure according to any of the preceding claims 7 to 9, wherein one of the following applies:
a. the first section has a maximum height that is equal to or larger than a maximum height of the first planar component; or
b. the first section has a maximum height that is equal to or less than a maximum height of the first planar component.

11. The structure according to any of the preceding claims 7 to 10, wherein the maximum height of the first section is xH, where H is the maximum height of the first planar component and x is in the range from 0.3 to 10.

12. The structure according to any of the preceding claims 7 to 11, wherein the maximum height of the first section is in the range from 0.4 to 14 mm.

13. The structure according to any of the preceding claims 7 to 12, wherein at least one or all of the following applies:
a. the maximum value of the first dimension of the first section is equal to or larger than the maximum value of the first dimension of the at least two openings of the first kind;
b. the maximum value of the first dimension of the first section is in the range 3 to 70 mm.

14. A method for transporting a plurality of containers, comprising the steps of
a. providing a structure according to any of the preceding claims;
b. receiving a plurality of containers in the structure;
c. preferably transporting the structure and the plurality of containers from a first location to a further location.

15. A use of the structure according to any of the preceding claims 1 to 13, for accommodating a plurality of containers, wherein said containers are for accommodating a pharmaceutical, medical and/or cosmetic composition.
